# EUROPEAN PATENT SPECIFICATION

(11) **EP 0 867 162 B1**
(45) Date of publication and mention of the grant of the patent: **04.09.2002**
(21) Application number: 97105063.8
(22) Date of filing: 25.03.1997
(51) Int. Cl.: A61F 13/00, A61F 13/04

(54) **Load removing walking cast**
Gewichtentlastender Laufgipsverband für den Unterschenkel
Plâtre de marche capable à enlever le poids pour la jambe inférieure

(43) Date of publication of application: 30.09.1998
(73) Proprietor: Castec Corporation, Kurume-shi, Fukuoka-ken (JP)
(72) Inventor: Goto, Takeshi, Kurume-shi, Fukuoka-ken (JP)
(74) Representative: Heusler, Wolfgang, Dipl.-Ing.

(56) References cited:
- CH-A- 273 249
- GB-A- 803 642
- US-A- 2 264 570
- US-A- 2 875 752
- US-A- 3 780 728

## Description

The present invention relates to lower leg load removing walking casts used for treatment of tibial fractures, that is, casts applied to an affected leg.

### DESCRIPTION OF RELATED ART

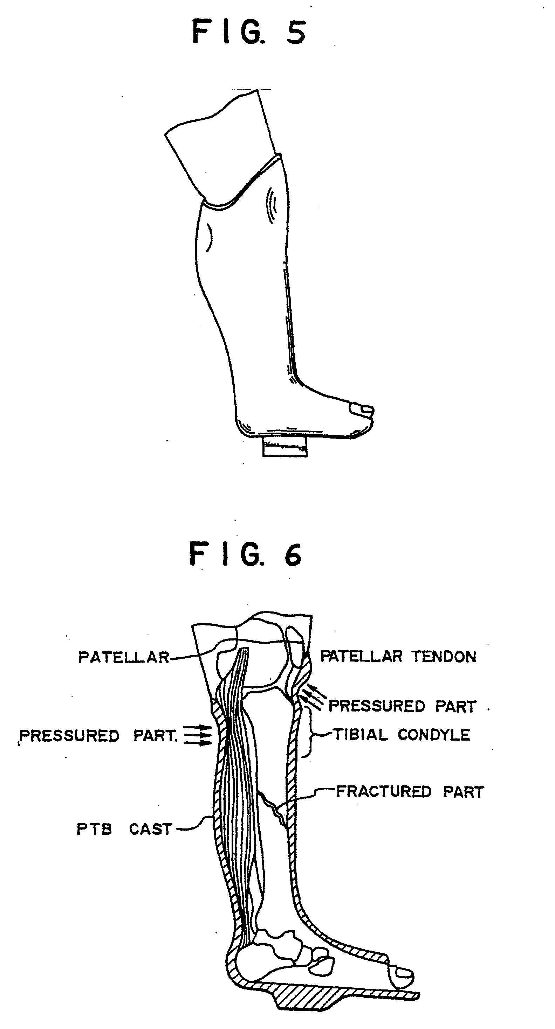

PTB casts (Patellar Tendon Bearing casts, so-called walking casts for lower leg) as seen in Fig.5 has broadly been employed to treat tibial fractures (fracture of leg bone). This cast is used for bearing a patient's human body weight at pressured parts around the knee joint (portions of the patellar tendon and tibial condyle) shown with arrows in Fig.6 in order to enable a knee joint to move or walk with a working cast after having treated the leg bone or at an early period after a surgical operation, thereby protecting the tibial fractured portion so as not to effect an excessive load thereon.

However, there are still many unknown matters in regard to the effects of removal of load which is significant purpose of the cast, and accordingly not a few of the medical specialist feel dubious about the load removing effect. These specialists allow patient to rely on crutches for assisting the load removal over a long period of days even after having worn the cast for assisting the load removal.

In view of the above-mentioned circumstances, the inventor and associates used a dynamic foot pressure analysis system which was first introduced in Japan in 1992 and measured the load removing effect of the cast, and made studies on the actual load removing effects. Then, the load removing effect brought about by the prior cast was about only 30% of the patient's human body weight. This data tells us that 70% of the patient's weight is, when walking, burdened as a load on his fractured leg, and it was found that the load removing effect by the above-described broadly used cast was unsatisfied.

In an early healing period while the tibial fracture is not yet recovered, if the body weight is given upon a fractured limb, and the load removing effect is insufficient, the load force by the body weight is added as a disadvantageous pressure to the fractured part which results in causing curing difficulties such as a shortening deformity of the fractured part or delaying a healing of the fracture. However, the theoretical idea to allow the knee joint to move or to allow control of the walking as loaded at an early recovering time at which the cast aims, is very important for healing the tibial fracture. Therefore, if the cast decreases or enables to control as theoretically the load by the body weight to the fractured part of the leg, it may be expected that the usefulness thereby will become greater. In this sense, the necessity to improve the load removing effect of the cast has been highlighted as a focus of attention.

Prior art lower leg casts are also known where the cast portion surrounding the lower leg is short and does not reach the area adjacent the knee of a patient and the rear part adjacent the knee (US-A 2,875,752). Such casts cannot result in reducing the loads applied to the lower leg, even if a space were formed between the foot and bottom of the cast during the preparation of the cast, since the leg is not suspended or supported by the cast adjacent the knee. Such casts are not effective to reduce the load on the lower leg and are not as effective as even the prior art PTB casts described above.

The reason why a satisfactory load removing effect could not be obtained by means of the existing cast of the type as shown in Figs. 5 and 6 is that since the bearing of the body weight by the cast is insufficient, the load moves, when burdened, in the burdening direction of the leg within the cast, and the foot is pushed up on its sole by the bottom of the cast. The inventor and associates made by test various embodiments of casts for a purpose of improving the load removing effect of the cast for the tibial fracture, and made appreciations and studies on the load removing effect by the dynamic foot pressure analysis system. When a test was prepared for a cast such that a predetermined space is disposed between the sole of the foot and the bottom of the cast, the foot was, during walking, moved or played along the length of a shin bone (in the loading direction of the body weight) in the cast, whereby the push-up by the bottom of the cast toward the sole of the foot could be prevented, and it was found that the load removing effect was largely improved. For easily and exactly comprehending the principle of improving the load removing effect of the PTB cast, the below-mentioned example should be considered.

Imagine a morning-glory or trumpet shaped instrument. If a hand is inserted into the instrument at a flared mouth thereof, the arm is held by a conical interior of the instrument and can no further move ahead. Herein, the trumpet shaped instrument corresponds to the cast, and the arm corresponds to the affected leg. A force making the hand go farther corresponds to a load by the body weight. Namely, with respect to the conventional cast for fractured leg, the prior art intended that the leg was supported in the interior cavity of the cast which was presumed as the hollow conical column. However, since the cast and the leg are very imperfect figures as conical bodies, a dynamic bearing power is limited in itself, and the leg somewhat slides within the cast due to the body weight toward the sole of the foot. This sliding of the leg causes a push-up to, or reactive force upon, the sole of the foot by the cast, and the push-up hampers the load removing effect. Therefore, by forming a space between the sole of the foot and the bottom of the cast, the factor of hampering the load removing effect can be taken away even if the leg slides within the cast. Thus, a satisfactory load removing effect can be made available.

Stated another way, the load removing lower leg cast of the prior art supports, at an upper portion of the lower leg part of the cast surrounding the entire part of the patient's lower leg, only a portion of the reactive forces caused by the patient's walking, and all of the remaining part of the loading forces are received at the interior bottom of the cast, bearing against the sole of a patient's foot, so that a satisfactory load removing effect could not be provided.

A walking cast according to the preamble of claim 1 is disclosed in GB-A 803 642.

According to the invention, the above explained problems are solveed by the characterizing features of claim 1.

In accordance with the present invention, the reactive loading force at the interior bottom of the cast is removed by the space between the sole of the foot and the bottom of the cast, and is also received at the upper portion of the lower leg cast in accordance with the morning-glory or trumpet theory mentioned above, thereby obtaining the effect of canceling the absolute total amount of the reactive loading forces acting upon the foot and the tibial fractured part, that is, a sufficient load removing effect is achieved. Further, when the size of the space is changed, the load removing effect may also be controlled.

Thus, the present invention has been devised on the basis of a new finding, wherein a casting plaster is surrounded around the patient's leg such that a predetermined space is formed between the sole of the foot and the bottom of the cast. The cast prepared by the invention has excellent load removing and walking effect.

The cast is formed with a predetermined space therebetween. If the patient walks with the leg cast, the foot can be moved or played in the space along the length of the shin bone (in the loading direction) within a predetermined range. This play absorbs an impact force or the load reactive force which would otherwise adversely influence the fractured bone of the leg, and thus a satisfactory load removing effect is made available.
Fig.1 shows a bellows bag that does not fall within the scope of the claims but serves to explain the invention. Fig.1(a) is a front view of this bellows bag, Fig.1(b) is a plan view of the same, and Fig.1(c) is a side view thereof.
Fig.2 shows the condition of the cast provided by the bellows bag of Fig.1 in use, in which Fig.2(a) is a front view of this condition, Fig.2(b) is a plan view of the same, and Fig.2(c) is a side view thereof.
Fig.3 shows the bellows bag of Fig.1, in which Fig.3(a) is a front view of this embodiment, Fig.3(b) is a plan of the same, and Fig.3 (c) is a side view thereof.
Fig.4 shows expansion and contraction of the bellows bag of Fig.1, in which Fig.4(a) is a case of the former, and Fig.4(b) is a case of the latter.
Fig.5 is an explanatory view showing a conventional leg cast (PTB cast).
Fig.6 shows parts aiming at supporting of a patient's human body weight (pressuring parts) in the above conventional leg cast (PTB cast), the supporting and pressurized portion of the cast also used in the above embodiments of the present invention.
Fig.7 shows a first embodiment in which Fig.7(a) is a side view of this embodiment, Fig.7(b) is a side view of the same, Fig.7(c) is a side view of the same, Fig.7(d) is a side view thereof, and Fig.7(e) is a side view thereof.
Fig.8 shows a second embodiment in which Fig.8(a) is a side view of this embodiment, and Fig.8(b) is a plan view of the same.
Fig.9 shows a third embodiment in which Fig.9(a) is a side view of this embodiment, Fig.9(b) is a plan view of the same and Fig.9(c) is a front view thereof; and
Fig.10 is a reference view.

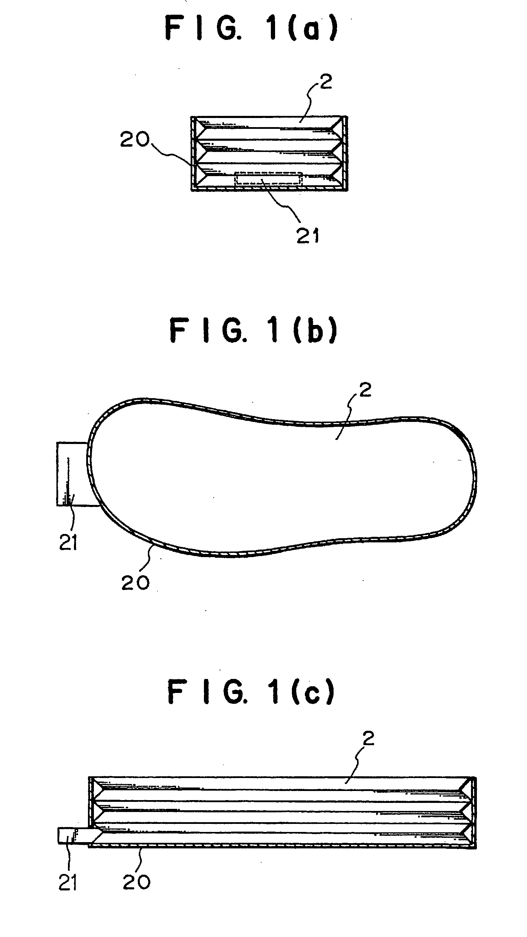

Reference will be made to Figs. 1-4 for explaining the steps of making the cast by means of the instruments exemplified herein.

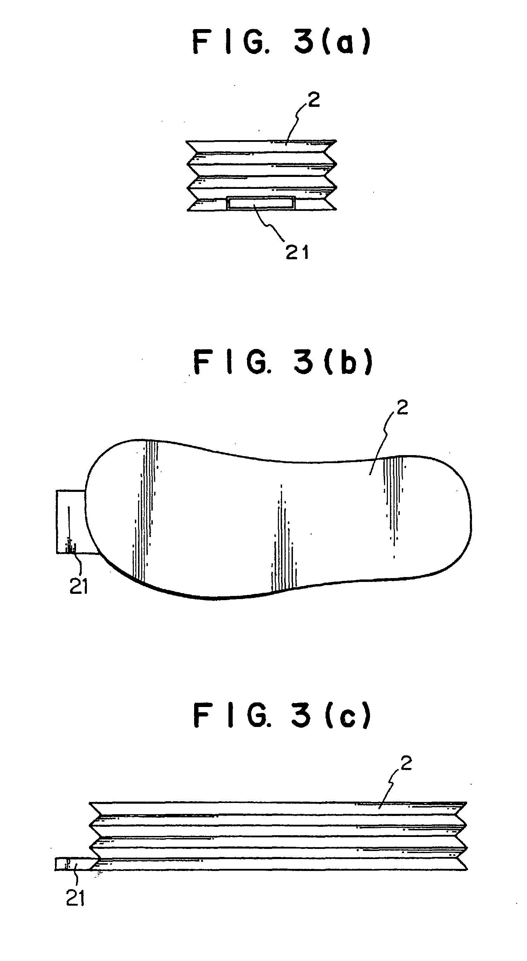

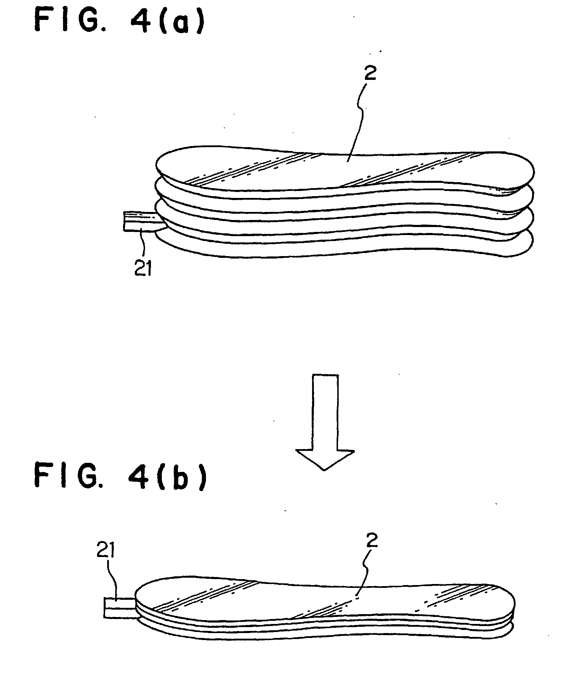

The bellows bag 2 is closed at an air hole 21 as seen in Fig.3 and is disposed within a protecting case 20 comprising a hard resin as illustrated in Fig.1. A foot 3 is then put on the bellows bag 2, followed by wrapping the casting plaster on the foot in an ordinary sequence and hardening it. When wrapping and hardening the casting plaster, a considerable pressure is burdened on the bellows bag 2, however it may perfectly maintain its shape against the pressuring force, since it is placed within the hard protecting case 20 and the air hole 21 is tightened or closed. When the air hole 21 is opened after the plaster 1 is solidified, the bellows bag 2 is made to freely expand and contract as is seen in Fig.4, and the load removing and walking cast having excellent load removing effect is accomplished.

The protecting case 20 serves to protect the soft and elastic bellows bag 2 against the cast 1 wrapped around the patient's leg, and due to this protecting service, the bellows bag 2 may expand and contract vertically (along the length of the shin bone) even by a weak pressure within the cast 1.

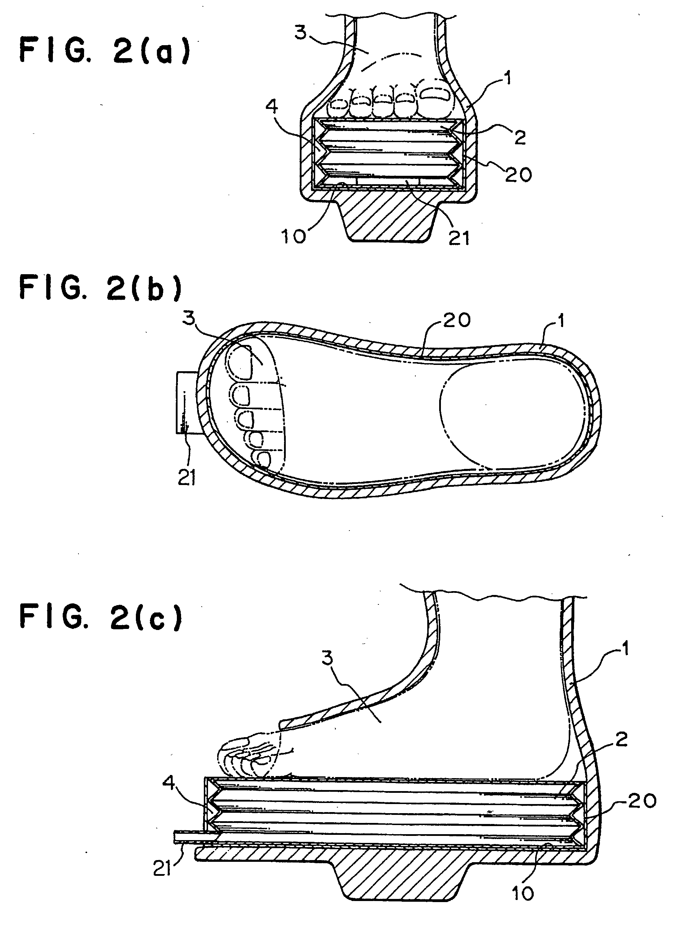

As shown in Fig.2, the space 4 exists between the sole of the foot 3 and the bottom 10 of the cast 1, and the protecting case 20 made of the hard synthetic resin is disposed in the space 4, within which the bellows bag 2 is placed. The bellows bag 2 communicates with the exterior through the air hole 21, and expands and contracts in response to moving of the foot as the patient walks as burdened. Therefore, when walking with the present cast 1, the bellows bag 2 placed in the space 4 is pressured and easily shrinked, and the foot 3 may moderately or slightly move along the length of the shin bone (in the loading direction) within the space 4, and this moving partially absorbs the loading force by the walking burden. Thus, as will be later exemplified, a sufficient load removing effect may be provided.

The load removing and walking cast 1 described above, and indeed the cast of each of the embodiments of the invention described herein, extends downward from the knee region, and is shaped at the knee region in a manner that catches around the knee and its adjacent parts. The leg of a patient is thus suspended by the shape of the cast with respect to the leg together with the pressure of the cast with respect to the leg adjacent the front and rear parts of the knee. That is, due to the shape of the cast and the catching or holding of the lower leg at the knee and the rear part of the knee by the cast, the leg is suspended and does not slide down, or slides down only slightly less than the distance of the space that is formed between the sole of the foot and the interior bottom portion of the cast. The suspension of the lower leg at the knee region in accordance with the teachings of the inventive cast as described, along with the maintaining of a sufficient space between the sole of a foot and the interior portion of the cast, is an important feature of the present invention to remove or substantially lessen the reactive forces caused by walking upon the lower leg.

The prior PTB cast of Figs.5 and 6 showed that the load removing effect was only 30.5% of the human body weight, while the load removing and walking effect of the present invention when the cast having the space distance of 1 cm was 55.9%, that in a case of 1.5 cm was 65.8%, that of 2.5 cm was 93.7%, and that of 3 cm was 100% which was the perfect load removing and walking effect.

As explaianed at length, the leg casts provided by means of the invention bring about remarkable results to largely improve the load removing and walking effect.

The sole of the foot does not contact the bottom due to suspending the lower leg by holding the lower leg at the knee and its adjacent parts, so that a space is maintained during walking. The reactive forces caused by walking are taken up through the cast and are at least partially borne at the upper portions of the lower leg adjacent the knee (front and rear), as previously described.

It should be appreciated that the cast of the present invention can extend above the knee and may extend to the hip. Such a cast may support the hip bone and provide load removal upon the entire leg including load removal at the thigh.

It may be appreciated that the embodiments using the elastic member provide for controlling the load removing effect. The elastic member which may vary the space, controls the load removing effect. That is, for healing the tibial fracture, a sufficient load removing effect is required immediately after suffering the tibial fracture. It is said that a callous appears around the fractured part as time goes on, and a moderate burden upon the fractured part advantageously acts on the bone formation. However, the prior art cast according to Fig.5 did not bring about a sufficient load removing effect required at the beginning period of curing the fractured bone, and, further, it was impossible to control the load removing effect in accordance with the curing progress. In contrast, in the present invention, as above exemplified, the load removing effect can be varied by changing the thickness of the elastic member. Therefore, if using the elastic members of different thicknesses in response to the curing progress, the load removing effect may be controlled, and it may be served as an optimum curing instrument.

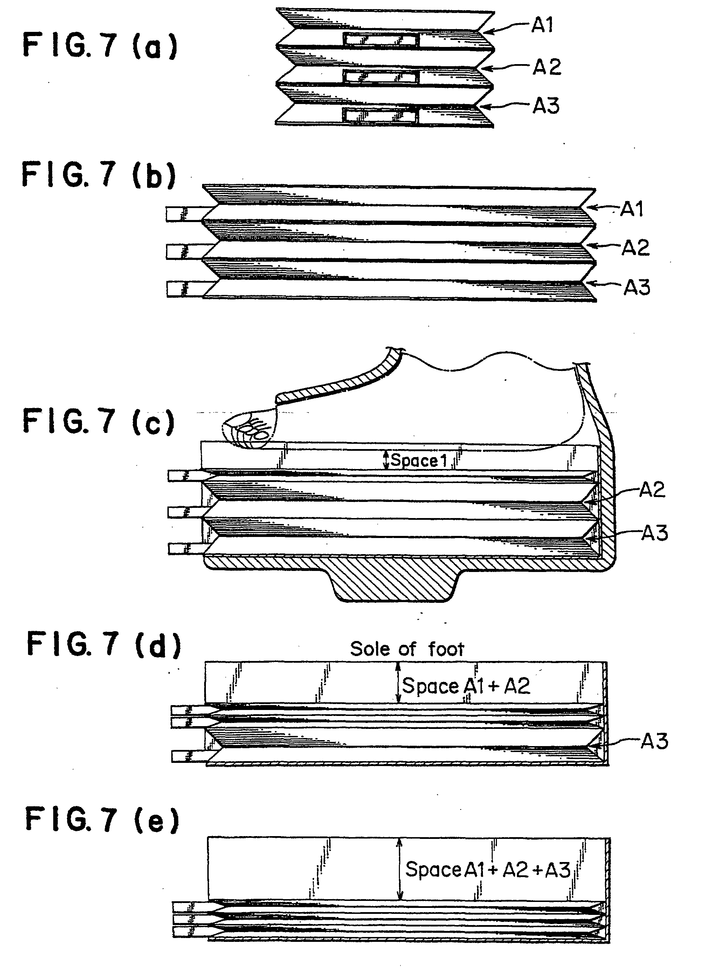

The present invention proposes bellows bags as illustrated in Figs. 7 to 10 as embodiments for controlling load removing effects. These embodiments constitute improvements of the bellows bags shown in Figs.1 to 4.

Basic principles or disposals of these embodiments within the cast are not different from those of the aforementioned bellows members, in which, however, the working function is more enhanced so that basically the foregoing bellows are divided into or are made two independent bellows parallel. Depending upon means of making the divided or parallelled embodiments, uses other than the above cases may be available as will be referred to below.

A first using embodiment is that the bellows is divided into a plurality of upper and lower independent spaces by means of interior isolating walls.

Fig.7 shows that the bellows is divided into independent three spaces A1, A2, A3 by the interior partitioning walls, and each of the spaces is equipped with a one air hole, whereby the bellows can be shrinked (shortened in width) by selectively operating the air hole, that is, the spaces for the foot's sliding are enlarged three stepwise. If the air hole is provided with a re-sealing stopper, an arbitrary space which has once been opened at the air hole and shrinked, is again expanded by introducing the air, so that the width of the space may be reduced.

In Fig.7, (a) and (b) show the bellows prior to opening the air hole, where the spaces A1, A2 and A3 are elongated due to the pressure of the enclosed air. (c) shows that the air hole of the space A1 is opened and only the space A1 is shrinked (shortened) to provide a space 1. Similarly, (d) shows that the air holes of the spaces A1, A2 are opened, and (e) shows that the air holes of the spaces A1, A2, A3 are opened, and the space A1+A2 and the space A1+A2+A3 are obtained, respectively.

If the air hole is provided with the re-sealing stopper, it is possible to return the shrinked bellows to an elongated state as a sequence of (e) → (d) → (c) → (b), and as a result such a load removing effect may be optionally provided alongside a curing progress while maintaining the bellows within the cast which does not require remaking.

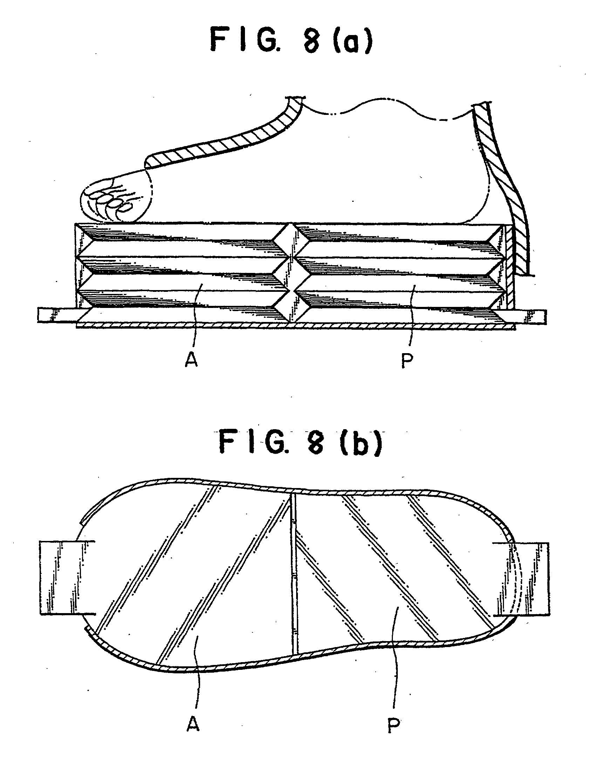

In a second embodiment illustrated in Fig. 8, the bellows are divided into two parts A and P of back and forth. Thus, side walls of the two bellows face one another therebetween, differently from the first embodiment. Since two bellows have individual air holes, each of them may be shortened or elongated indepedently.

The instant embodiment may be clinically used to bone fractures at front parts of foot or heel (rear part of foot). That is, if the bellows A of the front part is shortened, a load is burdened on the not shortened bellows P of the heel part, and thus the load is removed from the foot front part, and reversely if the rear bellows (the space) is shortened, the load is effected on the front part and the heel is removed from the load.

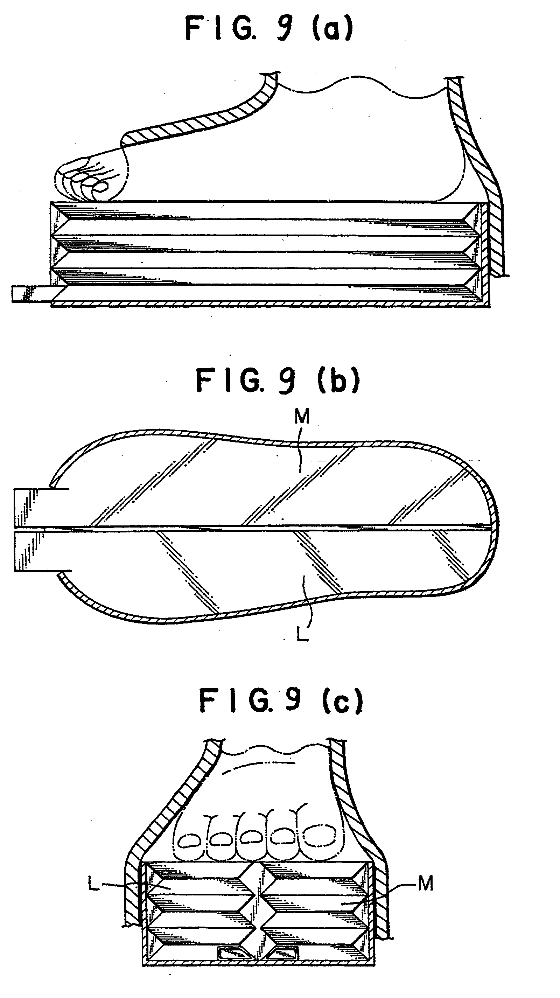

Fig.9 shows a third embodiment in which the bellows is divided at center longitudinally into independent right M and left L. The two bellows have independent air holes, and each of them may be shortened or elongated independently as the second embodiment does.

This embodiment is used when the inner part of the foot (i.e. a great toe) or the outer part (i.e. a little toe) is selectively effected with load removal. Using ways are similar to those of the second embodiment in that a part to be removed from load is shortened (shrinked).

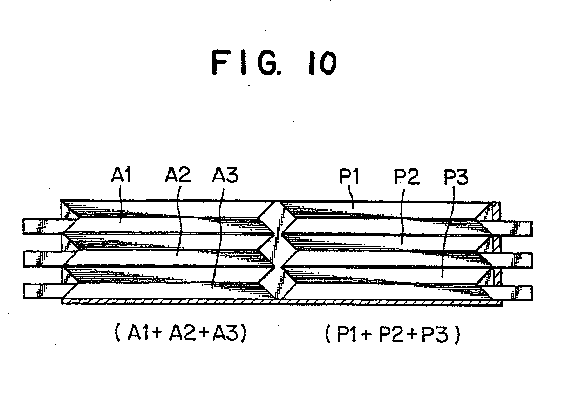

Fig.10 shows a fourth embodiment which is combined with the second embodiment or the first and third embodiments. Although a structure is complicated, the load may be removed per each of the independent bellows.

## Claims

1. A walking cast for surrounding at least the lower leg and foot of a patient, comprising:
a leg surrounding plaster cast portion for surrounding at least the patient's lower leg;
a plaster cast bottom portion including an interior bottom base, wherein a space is formed between the sole of the patient's foot and said interior bottom base,
bellows means extending within said space between the interior bottom base and the sole of the patient's foot;
**characterized by** said bellows means including at least two independent bellows chambers (A1, A2, A3) each provided with an air stopper at an air hole (21).

2. A walking cast as claimed in claim 1, wherein each of said chambers is formed by at least one bellows bag being divided into at least two independent chambers (A1, A2, A3) which are superimposed vertically and are separated by a partition.

3. A walking cast as claimed in claim 2, wherein said superimposed chambers (A1, A2, A3) of the bellows bag are three in number.

4. A walking cast as claimed in any one of the claims 1 to 3, wherein said bellows means has a center and is laterally divided into two parts (A, P) at its center.

5. A walking cast as claimed in any one of the claims 1 to 4, wherein said bellows means is longitudinally divided into two parts (M, L) along its length.

6. A walking cast as claimed in any one of the claims 1 to 5, wherein said bellows means (A1, A2, A3) is formed of soft resin.

7. A walking cast as claimed in any one of the claims 1 to 6,
wherein said bellows means (A1, A2, A3) is disposed within a space between a substantially planar base of a rigid protective casing (20) and the sole of the patient's foot,
and wherein said casing (20) lies upon said interior bottom base (10) of the cast and has sidewalls extending from the casing base and includes an opening adjacent said air holes of the bellows means to enable air passage therethrough.

## Patentansprüche

1. Gehgips, welcher mindestens den Unterschenkel und Fuß eines Patienten umgibt, mit
einem den Schenkel umgebenden Gipsbindenteil, der mindestens den Unterschenkel des Patienten umgibt,
einem Gipsbindenunterteil mit einer inneren Sohlenfläche, zwischen der und der Fußsohle des Patienten ein Zwischenraum gebildet wird,
einer Balganordnung, die in den Raum zwischen der inneren Sohlenfläche und der Fußsohle des Patienten ragt,
**dadurch gekennzeichnet, dass** die Balganordnung mindestens zwei unabhängige Balgkammern (A1,A2,A3) enthält, von denen jede mit einem Verschluss an einem Luftloch (21) versehen ist.

2. Gehgips nach Anspruch 1, bei welchem jede der Kammern durch mindestens einen Balg gebildet wird, der in mindestens zwei unabhängige Kammern (A1,A2,A3) unterteilt ist, die vertikal übereinander angeordnet und durch eine Trennwand voneinander getrennt sind.

3. Gehgips nach Anspruch 2, bei welchem der Balg (3) übereinanderliegende Kammern (A1,A2,A3) aufweist.

4. Gehgips nach einem der Ansprüche 1 bis 3, bei welchem die Balganordnung eine Mitte hat und an dieser seitlich in zwei Teile (A,P) unterteilt ist.

5. Gehgips nach einem der Ansprüche 1 bis 4, bei welchem die Balganordnung entlang ihrer Länge in zwei Teile (M,L) längs unterteilt ist.

6. Gehgips nach einem der Ansprüche 1 bis 5, bei welchem die Balganordnung (A1,A2,A3) aus weichem Harz gebildet ist.

7. Gehgips nach einem der Ansprüche 1 bis 6, bei welchem die Balganordnung (A1,A2,A3) innerhalb eines Raumes zwischen einer im wesentlichen ebenen Sohle eines festen Schutzgehäuses (20) und der Fußsohle des Patienten angeordnet ist,
und wobei das Gehäuse (20) auf der inneren Grundfläche (10) des Gipses aufsitzt und von der Gehäusesohle aufragende Seitenwände besitzt und neben den Luftlöchern der Balganordnung Öffnungen zum Luftdurchtritt enthält.

## Revendications

1. Plâtre de marche pour entourer au moins la jambe et le pied d'un patient, comprenant :
une partie de plâtre entourant la jambe pour entourer au moins la jambe du patient ;
une partie inférieure de plâtre comprenant une base inférieure intérieure, dans laquelle un espace est formé entre la plante du pied du patient et ladite base inférieure intérieure ;
des moyens formant soufflet s'étendant à l'intérieur dudit espace entre la base inférieure intérieure et la plante du pied du patient ;
**caractérisé en ce que** lesdits moyens formant soufflet comprennent au moins deux chambres à soufflet indépendantes (A1, A2, A3) chacune munie d'un bouchon à air et d'un trou à air (21).

2. Plâtre de marche selon la revendication 1, dans lequel chacune desdites chambres est formée par au moins un sac à soufflet étant divisé en au moins deux chambres indépendantes (A1, A2, A3) qui sont superposées verticalement et sont séparées par une séparation.

3. Plâtre de marche selon la revendication 2, dans lequel lesdites chambres superposées (A1, A2, A3) des sacs à soufflet sont au nombre de trois.

4. Plâtre de marche selon l'une quelconque des revendications 1 à 3, dans lequel lesdits moyens formant soufflet ont un centre et sont divisés latéralement en deux parties (A, P) en leur centre.

5. Plâtre de marche selon l'une quelconque des revendications 1 à 4, dans lequel lesdits moyens formant soufflet sont divisées longitudinalement en deux parties (M, L) le long de leur longueur.

6. Plâtre de marche selon l'une quelconque des revendications 1 à 5, dans lequel lesdits moyens formant soufflet (A1, A2, A3) sont formés de résine souple.

7. Plâtre de marche selon l'une quelconque des revendications 1 à 6,
dans lequel lesdits moyens formant soufflet (A1, A2, A3) sont disposés à l'intérieur d'un espace entre une base sensiblement plane d'un boîtier protecteur rigide (20) et la plante du pied du patient,
et dans lequel ledit boîtier (20) repose sur ladite base inférieure intérieure (10) du plâtre et a des parois de côtés s'étendant de la base du boîtier et comprend une ouverture adjacente aux dits trous à air des moyens formant soufflet pour permettre le passage de l'air à travers celle-ci.
